(19) Europäisches Patentamt
European Patent Office
Office européen des brevets

(11) EP 2 305 304 A1

(12) # EUROPEAN PATENT APPLICATION
published in accordance with Art. 153(4) EPC

(43) Date of publication:
**06.04.2011 Bulletin 2011/14**

(21) Application number: **09738800.3**

(22) Date of filing: **27.04.2009**

(51) Int Cl.:
***A61K 45/00*** (2006.01)
***A61K 38/00*** (2006.01)
***A61K 39/39*** (2006.01)
***A61P 31/00*** (2006.01)
***A61P 31/10*** (2006.01)
***A61P 35/00*** (2006.01)
***A61P 43/00*** (2006.01)
***A61K 31/5575*** (2006.01)
***A61K 39/00*** (2006.01)
***A61K 47/40*** (2006.01)
***A61P 31/04*** (2006.01)
***A61P 31/12*** (2006.01)
***A61P 37/04*** (2006.01)

(86) International application number:
**PCT/JP2009/058305**

(87) International publication number:
**WO 2009/133863 (05.11.2009 Gazette 2009/45)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **28.04.2008 JP 2008117825**

(71) Applicants:
- **National University Corporation Hamamatsu University School of Medicine Hamamatsu-shi, Shizuoka 431-3192 (JP)**
- **ONO Pharmaceutical Co., Ltd. Osaka-shi, Osaka 541-8526 (JP)**

(72) Inventors:
- **TAKIGAWA, Masahiro Hamamatsu-shi Shizuoka 431-3192 (JP)**
- **SEO, Naohiro Hamamatsu-shi Shizuoka 431-3192 (JP)**
- **MARUYAMA, Takayuki Mishima-gun Osaka 618-8585 (JP)**
- **KANAJI, Toshiya Mishima-gun Osaka 618-8585 (JP)**

(74) Representative: **Dean, John Paul et al Withers & Rogers LLP Goldings House 2 Hays Lane London SE1 2HW (GB)**

(54) **IMMUNOPOTENTIATING AGENT COMPRISING EP1 AGONIST**

(57) An EP1 agonist has an immunopotentiating effect mediated by cytotoxic T lymphocyte activation and/or natural killer cell activation, and is thus useful for the prevention and/or treatment of cancers, microbial infectious diseases and the like.



Printed by Jouve, 75001 PARIS (FR)

## Description

### TECHNICAL FIELD

The present invention relates to an immunopotentiating agent which includes an EP1 agonist. More specifically, the present invention relates to an EP1 agonist-containing immunopotentiating agent for use in immunopotentiation, particularly against cancers and/or microbial infectious diseases.

### BACKGROUND ART

Conventional modalities for treating cancer include surgical therapy, chemotherapy with anticancer drugs, radiation therapy, and therapeutic modalities representing a combination thereof. However, surgery is not fully effective in situations where cancer cells have metastasized, and a number of disadvantages are known to be associated with the use of chemotherapy and radiotherapy as adjuncts to surgery, including severe side effects arising from the destruction of normal cells as well as cancer cells, and resistance in the patient to anticancer drugs and radiation.

Investigations have recently been conducted on therapeutic methods for eliminating cancer cells by strengthening the body's own immune system (immunotherapy) as one new modality for treating cancer. For example, clinical studies using non-specific immunostimulants (e.g., Krestin, Bestatin), cytokine therapies (e.g., interferons, interleukins), antibody therapies (e.g., herceptin), and immune cell therapies (e.g., dendritic cell vaccine therapy, peptide vaccine therapy) are being carried out. In particular, in an immune cell therapy aimed at activating cytotoxic T lymphocytes (CTL) that has recently been attracting attention, immature T cells recognize a complex of a major histocompatibility complex (MHC) molecule and an antigen peptide (e.g., a fragmented cancer cell antigen) that is presented by antigen-presenting cells (e.g., dendritic cells, macrophages); at the same time, a signal comes from a co-stimulatory molecule, inducing the immature T cells to develop into mature T cells having a cytotoxicity (CTL) specific to the presented complex. Dendritic cell vaccine therapy efficiently induces CTLs by administering cultured antigen-presenting dendritic cells, but problems relating to the use of a culture broth containing fetal calf serum and the route of administration are of concern. In peptide vaccine therapy, an antigen peptide derived from inactivated cancer cells or microorganism-infected cells is administered to promote the activation of CTLs that specifically recognize the antigen. Moreover, by using microbial peptides such as inactivated virus fragments or protozoan peptides as the antigen peptide, immune cell therapies are being applied not only to cancers, but also to microbial infectious diseases.

Safer and more efficient immunotherapies have hitherto been developed by combining the above. For example, a method of transcutaneous immunotherapy has been developed wherein the corneal layer of the epidermis is removed using highly adhesive tape to activate epidermal Langerhans cells (a type of dendritic cell), and antigen peptides are applied to the skin thus deprived of the corneal layer to induce CTLs in vivo (see Patent Document 1 and Non-patent Documents 1 and 2).

It is also known that EP1 agonists, which specifically bind to EP1 receptors, a sub-type of PGE2 receptor, allow malignant keratinocytes to proliferate (see Non-patent Document 3), and induce the differentiation of undifferentiated T cells to Th1 cells (see Non-patent Document 4). In addition, EP1 antagonists are known to be effective in the treatment of intestinal cancer and breast cancer (see Patent Document 2).

However, these documents do not in any way state or suggest that EP1 agonists have an immunopotentiating effect mediated by cytotoxic T lymphocyte activation and/or natural killer cell activation, particularly that EP1 agonists have an immunopotentiating action against cancers (particularly melanoma) and viral infectious diseases (particularly influenza viruses).

Patent Document 1: Japanese Patent No. 3879785
Patent Document 2: WO 00/69465


Non-patent Document 1: Proceedings of the Natural Academy Sciences of USA, Vol. 97, pp. 371-376 (2000).
Non-patent Document 2: Cancer Research, Vol. 66, pp. 10136-10144 (2006).
Non-patent Document 3: Neoplasia, Vol. 3, pp. 402-410 (2001).
Non-patent Document 4: The Journal of Experimental Medicine, Vol. 204, p. 2865-2874 (2007).


### DISCLOSURE OF THE INVENTION

Attempts have hitherto been made to employ immune cell therapies as methods for treating cancers and

infectious diseases. However, because of difficulties having to do with efficacy and methods for administering the antigen peptides used in such therapies and owing also to problems with the safety of dendritic cells, immune cell therapies have yet to be established as effective modes of treatment. Accordingly, it is an object of the present invention to provide a substance of a low molecular weight and higher safety that can be easily administered, which substance has an immunopotentiating action against cancers and viruses which is mediated by cytotoxic T lymphocyte activation and/or natural killer cell activation, and can also be used as an adjuvant.

It is therefore an object of the present invention to provide a substance of low molecular weight and capable of easier administration, which substance has an immunopotentiating effect against cancers and viruses, and can also be used as an adjuvant.

The inventors have conducted extensive investigations in order to achieve the above object. As a result, they have discovered the surprising facts that EP1 agonists: (1) have an immunopotentiating effect, and particularly (2) have an immunopotentiating effect against cancers and/or microbial infectious diseases.

That is, the present invention relates to:

[1] an immunopotentiating agent comprising an EP1 agonist,

[2] the agent of [1] above, wherein the EP1 agonist is a 6-oxo-PGE$_1$ compound represented by general formula (I)

[C1]

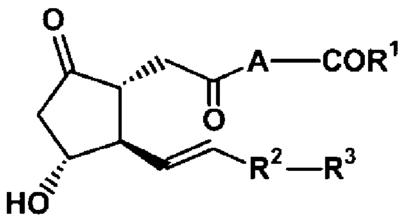

(I)

(wherein A is a 4-7 membered carbon ring;
$R^1$ is hydroxyl, $C_{1-4}$ alkoxy or $NR^4R^5$ ($R^4$ and $R^5$ being each independently a hydrogen atom or $C_{1-4}$ alkyl);
$R^2$ is $C_{1-8}$ alkylene, $C_{3-8}$ alkenylene or $C_{3-8}$ alkynylene substituted with one hydroxyl;
$R^3$ is

(1) a hydrogen atom or $C_{1-4}$ alkyl,
(2) phenyl or $C_{3-7}$ cycloalkyl which may be substituted with 1 to 3 substituents selected from among $C_{1-4}$ alkyl, $C_{1-4}$ alkoxy, halogen atoms, trifluoromethyl and nitro, or
(3) phenoxy which may be substituted with 1 to 3 substituents selected from among $C_{1-4}$ alkyl, $C_{1-4}$ alkoxy, halogen atoms, trifluoromethyl and nitro;

[C2]

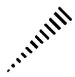

is an α-configuration bond; and

[C3]

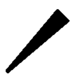

is a β-configuration bond,
with the proviso that (i) when $R^2$ is a $C_{3-8}$ alkenylene or $C_{3-8}$ alkynylene substituted with one hydroxyl, the hydroxyl is not bonded to a carbon atom associated with a double bond or triple bond, and (ii) when $R^3$ is (3),

the hydroxyl on $R^2$ and the $R^3$ radical are not bonded to the same carbon atom), or a salt or cyclodextrin clathrate thereof,

[3] the agent of [1] above, wherein the agent is used for immunopotentiation against a cancer and/or a microbial infectious disease,
[4] the agent of [3] above, wherein the cancer is one or more selected from among a digestive organ cancer, a skin cancer, a respiratory cancer, a urogenital cancer, a liver cancer and a pancreatic cancer,
[5] the agent of [4] above, wherein the skin cancer is melanoma,
[6] the agent of [3] above, wherein the microorganism is one or more selected from among a virus, a bacterium and a fungus,
[7] the agent of [6] above, wherein the virus is an influenza virus,
[8] the agent of [2] above, wherein the EP1 agonist is (13E)-(11$\alpha$,15S,17S)-2,5-ethano-6,9-dioxo-11,15-dihydroxy-17,20-dimethylprosta-13-enoic acid,
[9] the agent of [1] above, further comprising an antigen peptide,
[10] the agent of [8] above, wherein the antigen peptide is a melanoma-specific antigen peptide,
[11] a pharmaceutical for potentiating immunity to melanoma, comprising (13E)-(11$\alpha$,15S,17S)-2,5-ethano-6,9-dioxo-11,15-dihydroxy-17,20-dimethylprosta-13-enoic acid as an active ingredient and optionally including a melanoma-specific antigen peptide,
[12] a method of immunopotentiation in a mammal, comprising administering an effective dose of an EP1 agonist to the mammal in optionalcombination with an antigen peptide,
[13] use of an EP1 agonist for producing an immunopotentiating agent, wherein the EP1 agonist is optionally used in combination with an antigen peptide, and
[14] an EP1 agonist for immunopotentiation, wherein the EP1 agonist is optionally used in combination with an antigen peptide.

EP1 agonists have an immunopotentiating effect mediated by cytotoxic T lymphocyte activation and/or natural killer cell activation, and are thus useful for preventing and/or treating cancer, microbial infectious diseases and the like. When used concomitantly with antigen peptides, effects as an adjuvant can also be obtained.

## BRIEF DESCRIPTION OF THE DRAWINGS

FIG. 1 is a graph showing the melanoma proliferation-inhibiting effects in Experimental Groups (1), (2) and (3) of Example 4.

## BEST MODE FOR CARRYING OUT THE INVENTION

### Immunopotentiating agent

In the present invention, as shown in the examples below, EP1 agonists have by themselves a cytotoxic T lymphocyte (also abbreviated below as CTL) activating and/or natural killer cell activating effect, enabling them to be used alone as an immunopotentiating agent.
In the present invention, immunopotentiating agent refers to a drug having an immunopotentiating effect. Here, immunopotentiation (or immunopotentiating effect) refers to a strengthening in the effect of eliminating target cells infected by invasive agents (e.g., cancer cell, pathogenic microorganisms), based on the activation of cytotoxic T lymphocytes and/or natural killer cells (NK1.1+ cells). Examples of diseases that can be prevented and/or cured by such immunopotentiation include cancers and microbial infectious diseases.
In this invention, cancers that can be prevented and/or treated by immunopotentiation include all of what are generally called malignant tumors. Examples include cancers involving cranial nerves (such as pediatric brain tumors (e.g., neuroblastoma, medulloblastoma, astrocytoma (juvenile piloid astrocytoma), ependymoma, craniopharyngeoma, germinoma, optic nerve glioma, choroid plexus papilloma, brain stem glioma), adult brain tumors (e.g., adult astrocytoma, adult malignant astrocytoma, adult glioblastoma, adult cerebral ventricular ependymoma, adult malignant cerebral ventricular ependymoma, adult malignant oligodendrocytoma, adult medulloblastoma, adult meningioma, adult malignant meningioma), gliomas (e.g., astrocytoma, oligodendroglioma, ependymoma, brain stem glioma), pituitary adenoma, acoustic schwannoma, retinoblastoma and uveal malignant melanoma), cancers of the respiratory organs (such as pharyngeal cancers (e.g., epipharyngeal cancer, mesopharyngeal cancer, hypopharyngeal cancer), laryngeal cancer, paranasal sinus cancer, lung cancers (e.g., small-cell cancer, non-small-cell cancer), thymoma and mesothelioma), cancers of the digestive organs (such as esophageal cancer, gastric cancer, duodenal cancer, and colorectal cancers (e.g., colic cancer, rectal cancer, anal cancer)), oral cancers (e.g., gingival cancer, lingual cancer, salivary gland cancer),

urogenital cancers (e.g., penile cancer, pelvic-urethral cancer, renal cell carcinoma, testicular (orchis) tumor, prostatic cancer, urinary bladder cancer), femalespecific cancers (such as vulvar cancer, uterine cancers (e.g., uterine cervical cancer, uterine body cancer (uterine intimal cancer)), uterine sarcoma, villous diseases (e.g., hydatid moles, choriocarcinoma, placental villous tumors, persistent trophoblastic disease), vaginal cancer, breast cancer, breast sarcoma, ovarian cancer and ovarian germinoma), skin cancers (such as melanomas (malignant melanomas) (e.g., malignant lentigo melanoma, superficial spreading melanoma, nodular melanoma, acral lentigenous melanoma, erosive melanoma), mycosis fungoides, squamous cell carcinoma, basal cell carcinoma, skin cancer prodromes/intraepidermal carcinomas (e.g., actinic keratosis, Bowen's disease, Paget's disease), lymphomatoid papulosis, dermal CD30-positive anaplastic large-cell lymphoma, Sézary syndrome, dermal B cell lymphoma), bone/muscular cancers (e.g., osteosarcoma, sarcoma of soft parts, rhabdomyosarcoma, synovial sarcoma, liposarcoma), thyroid cancer, carcinoid, liver cancer (hepatoma), hepatoblastoma, cholangioma, gall bladder cancer, pancreatic cancer, pancreatic endocrine tumors (e.g., insulinoma, gastrinoma, VIP-producing adenoma), cancers of unknown origin, hereditary tumors/familial tumors (e.g., hereditary non-polyposis colorectal cancer, familial colorectal polyposis, hereditary breast cancer, ovarian cancer syndrome, Li-Fraumeni syndrome, hereditary melanoma, Wilms' tumor, hereditary papillary renal cell carcinoma, von Hippel-Lindau syndrome, multiple endocrine oncosis), leukemias (e.g., acute myelocytic leukemia, acute lymphocytic leukemia, osteomyelodysplasia syndrome, chronic myelocytic leukemia/chronic myeloproliferative disease, adult T cell leukemia lymphoma, chronic lymphocytic leukemia/small-cell lymphoma), multiple myeloma, primary macroglobulinemia, and malignant lymphomas (e.g., Hodgkin's lymphoma, moderately or highly malignant lymphoma, Burkitt's lymphoma, lymphoblastic lymphoma, follicular lymphoma, mantle cell lymphoma, MALT (Mucosa-Associated Lymphoid Tissue) lymphoma, NK (natural killer) cell lymphoma). Preferred examples of cancers that can be prevented and/or treated by immunopotentiation include cancers of the digestive organs, skin cancers, cancers of the respiratory organs, urogenital cancers, liver cancers, and pancreatic cancers. Skin cancers are more preferred, and melanomas are especially preferred.

In the present invention, microbial infectious diseases that may be prevented and/or treated by immunopotentiation include all of what are generally called infectious diseases, and are specifically exemplified by infections that arise as a result of the proliferation of normal cells in the body that have been infected by one or more type of pathogenic microorganism, such as viruses, bacteria and fungi. Such pathogenic microorganisms also include rickettsia, chlamydia, protozoans and parasites.

In the present invention, viruses involved in microbial infectious diseases are exemplified by human hepatitis viruses (e.g., hepatitis B, hepatitis C, hepatitis A, hepatitis E), human retroviruses, human immunodeficiency viruses (e.g., HIV1, HIV2), human T cell leukemia viruses or human T lymph-oriented viruses (e.g., HTLV1, HTLV2), herpes simplex virus type 1 or type 2, Epstein-Barr (EB) virus, cytomegalovirus, varicella-zoster virus, human herpes viruses (e.g., human herpes virus 6), poliovirus, measles virus, rubella virus, Japanese encephalitis virus, mumps virus, influenza viruses, cold viruses (e.g., adenoviruses, enteroviruses, rhinoviruses), viruses that cause severe acute respiratory syndromes (SARS), Ebola virus, West Nile virus, flavivirus, echovirus, Coxsackie virus, coronavirus, respiratory coenocytic (syncytial) virus, rotaviruses, noroviruses, sapoviruses, measles virus, Parvovirus, vaccinia virus, HTL virus, dengue virus, papilloma virus, molluscum virus, rabies virus, JC virus, arbovirus, encephalitis viruses and hantavirus. Preferred examples of viruses involved in microbial infectious diseases capable of being prevented and/or treated by immunopotentiation include influenza viruses.

In the present invention, examples of influenza viruses include the influenza A virus, the influenza B virus and the influenza C virus. Subtypes of the influenza A virus include H1N1, H2N2, H2N8, H3N2, H3N8, H5N1, H7N6 and H9N2. Here, "subtype" refers to groups classified according to, of the surface antigens on the virus, differences in the respective antigenicities of 9 hemagglutinin (HA) antigens and 15 neuraminidase (NA) antigens, and differences in combinations of both types of antigen. The infectious host (e.g., human, avian) for the influenza type A virus in this invention is not subject to any limitation.

In the present invention, bacteria involved in microbial infectious diseases are exemplified by *vibrio* cholerae, Salmonella bacteria, *Escherichia coli,* Legionella bacteria, *Bacillus anthracis, Helicobacter pylori, Listeria monocytogenes,* tubercle bacilli, non-tuberculous acid-fast bacteria, staphylococci, streptococci, pneumococci, *Neisseria meningitidis,* pneumobacilli, Serratia bacteria, *Corynebacterium* diphtheriae, brucellae, *Bartonella henselae, Erysipelothrix* rhusiopathiae, actinomycetes, Lyme disease Borrelia, *Clostridium perfringens,* dysentery bacilli, *Yersinia pestis, Clostridium tetani* and Enterobacter bacteria.

In the present invention, fungi involved in microbial infectious diseases are exemplified by Candida, Aspergillus, Cryptococcus, Blastomyces, Coccidioides, Histoplasma, Paracoccidioides and Sporothrix.

In the present invention, examples of protozoans involved in microbial infectious diseases include malaria protozoans and toxoplasma protozoans.

In the present invention, examples of parasites involved in microbial infectious diseases include dysenteric ameba, ascarids, Babesia, Cryptosporidium, *Giardia lamblia,* uncinaria, oxyuris, schistosomes, taeniid, trichinae and trichuris.

In the present invention, examples of other microorganisms involved in microbial infectious diseases include mycoplasmata and spirochetes.

EP1 Agonist

The immunopotentiating agent according to the present invention (sometimes referred to below as simply the agent of the present invention) is an immunopotentiating agent which includes an EP1 agonist. EP1 agonists which may be used in this invention include any compound which selectively bonds to EP1, a subtype of prostaglandin E2 (PGE2) receptor, and exhibits an agonist activity. Such EP1 agonists include, in addition to those which are known at present, those which will be discovered in the future. EP1 agonists known at present are exemplified by the compounds mentioned in (A) to (D) below.

(A) Compounds represented by general formula (I) below are noted in Japanese Patent Application Laid-open No. H11-322709 as having an EP1 agonist activity. Definitions of each radical on the compound shown in general formula (I) are described in detail in Japanese Patent Application Laid-open No. H11-322709. Accordingly, EP1 agonists which may be used in the agent of the present invention include 6-oxo-$PGE_1$ compounds represented by general formula (I)

[C4]

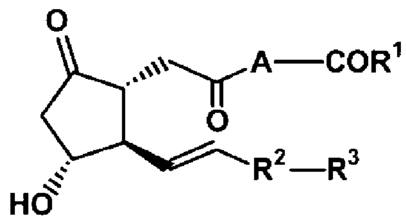

(I)

(wherein A is a 4-7 membered carbon ring;
$R^1$ is hydroxyl, $C_{1-4}$ alkoxy or $NR^4R^5$ ($R^4$ and $R^5$ being each independently a hydrogen atom or $C_{1-4}$ alkyl);
$R^2$ is $C_{1-8}$ alkylene, $C_{3-8}$ alkenylene or $C_{3-8}$ alkynylene substituted with one hydroxyl;
$R^3$ is

(1) a hydrogen atom or $C_{1-4}$ alkyl,
(2) phenyl or $C_{3-7}$ cycloalkyl which may be substituted with 1 to 3 substituents selected from among $C_{1-4}$ alkyl, $C_{1-4}$ alkoxy, halogen atoms, trifluoromethyl and nitro, or
(3) phenoxy which may be substituted with 1 to 3 substituents selected from among $C_{1-4}$ alkyl, $C_{1-4}$ alkoxy, halogen atoms, trifluoromethyl and nitro;

[C5]

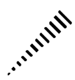

is an α-configuration bond; and

[C6]

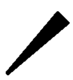

is a β-configuration bond,
with the proviso that (i) when $R^2$ is a $C_{3-8}$ alkenylene or $C_{3-8}$ alkynylene substituted with one hydroxyl, the hydroxyl

is not bonded to a carbon atom associated with a double bond or triple bond, and (ii) when $R^3$ is (3), the hydroxyl on $R^2$ and the $R^3$ radical are not bonded to the same carbon atom), or a salt or cyclodextrin clathrate thereof.

(B) WO 99/02165 mentions that compounds represented by general formula (IB) below have an EP1 agonist activity. Definitions of each radical on the compound shown in general formula (IB) are described in detail in WO 99/02165. Accordingly, EP1 agonists which may be used in the agent of the present invention include prostaglandin derivatives represented by general formula (IB)

[C7]

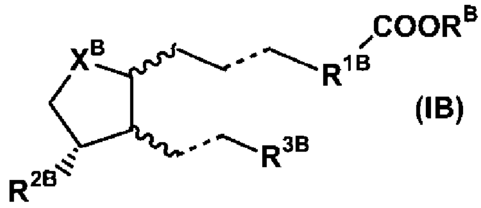

(IB)

(wherein bonds indicated by wavy lines represent an α or β structure; bonds indicated by dashed lines represent a single bond, a triple bond, or a double bond having a cis or trans structure;
$R^B$ is hydrogen, saturated or unsaturated alkyl (preferably $C_{1-10}$ alkyl), cycloalkyl (preferably $C_{3-8}$ cycloalkyl), aryl, arylalkyl (preferably aryl-$C_{2-5}$ alkyl), or heteroaryl;
$R^{1B}$ is saturated or unsaturated alkyl or cycloalkyl (preferably $C_{3-7}$ cycloalkyl) having 2 to 5 carbon atoms with an optionally intervening heteroatom selected from among oxygen, sulfur and nitrogen, cycloalkenyl (preferably $C_{3-7}$ cycloalkenyl), aryl or heteroaryl;
$X^B$ is C-OH or C=O;
$R^{2B}$ is hydrogen, hydroxyl, methyl, ethyl, methoxy or $OCOR^{4B}$ ($R^{4B}$ being a linear or branched, saturated or unsaturated alkyl (preferably $C_{1-10}$ alkyl, and more preferably $C_{1-6}$ alkyl), cycloalkyl (preferably $C_{3-8}$ cycloalkyl) or aryl); and
$R^{3B}$ is a linear or branched, saturated or unsaturated alkyl of preferably 3 to 8 carbon atoms, and more preferably 3 to 5 carbon atoms, having one or more optionally intervening heteroatom selected from among oxygen, sulfur and nitrogen, wherein each carbon atom is optionally substituted with a substituent selected from among $C_{1-6}$ alkyl, hydroxyl and carbonyl, hydroxyl and carbonyl being preferentially bonded to the position 15 carbon of the prostaglandin structure and the alkyl being capable of substitution by a single substituent, or independently by a plurality of substituents, selected from among $C_{1-3}$ alkyl, $C_{1-3}$ alkoxy, hydroxyl, nitro, trifluoromethyl and halogen, and optionally including cycloalkyl (preferably $C_{3-8}$ cycloalkyl), aryl or heteroaryl),
or a pharmaceutically acceptable salt or ester thereof.

(C) WO 00/51585 mentions that compounds represented by general formula (IC) below have an EP1 agonist activity. Definitions of each radical on the compound shown in general formula (IC) are described in detail in WO 00/51585. Accordingly, EP1 agonists which may be used in the agent of the present invention include prostaglandin analogs represented by general formula (IC)

[C8]

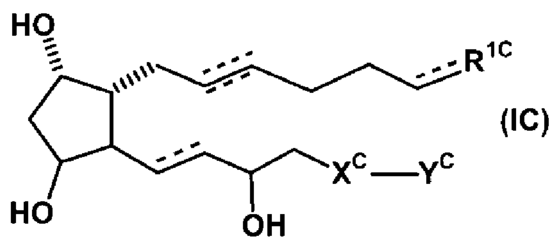

(IC)

(wherein
$R^{1C}$ is $CO_2H$, C(O)NHOH, $CO_2R^{2C}$, $CH_2OH$, $S(O)_2R^{2C}$, $C(O)NHR^{2C}$, $C(O)NHS(O)_2R^{2C}$ or tetrazole;
$R^{2C}$ is alkyl, heteroalkyl, a carbocyclic aliphatic ring, a heterocyclic aliphatic ring, an aromatic ring or a heterocyclic aromatic ring;
$X^C$ is $(CH_2)_{nC}$ (where nC is an integer from 0 to 3), NH, S or O; and
$Y^C$ a cycloalkyl or aromatic moiety, either substituted or unsubstituted.).

(D) WO 00/51616 mentions that compounds represented by general formula (ID) below have an EP1 agonist activity. Definitions of each radical on the compound shown in general formula (ID) are described in detail in WO 00/51616. Accordingly, EP1 agonists which may be used in the agent of the present invention include prostaglandin analogs represented by general formula (ID)

[C9]

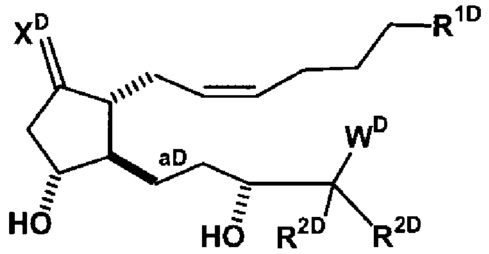

(ID)

(wherein
$R^{1D}$ is $CO_2H$, C(O)NHOH, $CO_2R^{3D}$, $CH_2OH$, $S(O)_2R^{3D}$ or $C(O)NHR^{3D}$ ($R^{3D}$ being independently alkyl, heteroalkyl, a carboxylic aliphatic ring, a heterocyclic aliphatic ring, an aromatic ring or a heterocyclic aromatic ring);
$X^D$ is $CH_2$, O or N-$OR^{4D}$ ($R^{4D}$ being hydrogen or lower alkyl); aD is a single bond, a trans double bond or a triple bond; each $R^{2D}$ is independently hydrogen or lower alkyl; and $W^D$ is (a) $[C(R^{5D})(R^{5D})]_{mD}$-$Y^D$-$[C(R^{5D})(R^{5D})]_{nD}$-$Z^D$ (wherein $R^{5D}$ in each instance is independently hydrogen, lower alkyl, alkoxy or halogen; mD is an integer from 0 to 1; nD is an integer from 0 to 1; $Y^D$ is $C(R^{5D})(R^{5D})$, O, NH, S or a covalent bond; and $Z^D$ is phenyl, thienyl, or furanyl, which phenyl, thienyl or furanyl may be unsubstituted or substituted with 1 or 2 halogens) or (b) $[C(R^{5D})(R^{5D})]_{pD}$-$U^D$-$[C(R^{5D})(R^{5D})]_{qD}$ (wherein $R^{5D}$ is the same as above; pD is an integer from 1 to 3 and qD is an integer from 1 to 3, such that pD+qD is from 1 to 4; and $U^D$ is $C(R^{5D})(R^{5D})$, O, NH or S).

The EP1 agonist used in the agent of the present invention is preferably a compound represented by general formula (I), and more preferably is selected from among (13E)-(11α, 15S,17S)-2,5-ethano-6,9-dioxo-11,15-dihydroxy-17,20-dimethylprosta-13-enoic acid and sulprostone.

The agent of the present invention may include any one of the various above-described types of EP1 agonists, or may include together two or more such types in any combination and ratio.

Isomers

In cases where the compounds of above general formula (I) and general formulas (IB) to (ID) (sometimes abbreviated below as compounds that may be used in the agent of the present invention) have isomers, unless otherwise specified, all isomers of these compounds are encompassed by the present invention. For example, alkyl, alkenyl, alkynyl, alkyloxy, alkoxy, alkenyloxy, alkynyloxy, alkylthio, alkylsulfinyl, alkylsulfonyl, alkylene, alkenylene, alkynylene, acyl and acyloxy groups include those which are linear and those which are branched. In addition, isomers at double bonds, rings and condensed rings (E-, Z-, cis-, and trans-isomers), isomers generated from asymmetric carbons (R- and S-isomers, α- and β-configurations, enantiomers, diastereomers), optically active isomers having optical rotation (D-, L-, d-, and ℓ-isomers), polar isomers obtained by chromatographic separation (high-polarity isomers, low polarity isomers), equilibrium compounds, rotational isomers, and mixtures or racemic mixtures thereof in any proportion are all encompassed by the invention. The present invention also encompasses all tautomers.

Salts and Solvates

The salts of the compound used in the agent of the present invention include all pharmaceutically acceptable salts. Pharmaceutically acceptable salts are preferably salts which have a low toxicity and are water-soluble. Illustrative

examples of suitable salts include salts of alkali metals (e.g., potassium, sodium, lithium), salts of alkaline earth metals (e.g., calcium, magnesium), ammonium salts (e.g., tetramethylammonium salts, tetrabutylammonium salts), salts of organoamines (e.g., triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine), acid addition salts (e.g., inorganic salts (e.g., hydrochlorides, hydrobromides, hydroiodides, sulfates, phosphates, nitrates) and organic acid salts (e.g., acetates, trifluoroacetates, lactates, tartrates, oxalates, fumarates, maleates, benzoates, citrates, methanesulfonates, ethanesulfonates, benzenesulfonates, toluenesulfonates, isethionates, glucuronates, gluconates)) .

Additional salts include quaternary ammonium salts. Quaternary ammonium salt refers to a compound wherein the nitrogen atom of the compound used in the agent of the present invention is quaternized by $R^0$ groups. $R^0$ groups are $C_{1-8}$ alkyl groups which may be substituted with phenyl.

Suitable solvates of the compound used in the agent of the present invention are exemplified by solvates of water and alcoholic solvents (e.g., methanol, ethanol). The solvate preferably has a low toxicity, and water solubility. Solvates of the compound used in the agent of the present invention include alkali (or alkaline earth) metal salts, ammonium salts, organic amine salts and acid addition salts of the compound.

The compound used in the agent of the present invention may be converted to the above salts or above solvates by a known method.

Cyclodextrin Clathrate

The compound used in the present invention may be converted to a cyclodextrin clathrate by the method described in Japanese Patent Publication No. S50-3362, S52-31404 or S61-52146 using α-, β- or γ-cyclodextrin, or a mixture thereof. Conversion to a cyclodextrin clathrate enhances the safety and increases the water solubility, which is desirable for use as a pharmaceutical. Of the above, conversion to an α-cyclodextrin clathrate is preferred.

Prodrug

A prodrug of the compound used in the agent of the present invention refers to a compound which is converted in vivo by a reaction involving an enzyme, gastric acid or the like into the drug used in the agent of the present invention. Prodrugs of the compound used in the agent of the present invention are exemplified by, in cases where the compound used in the agent of the present invention includes an amino group, compounds in which the amino group has been acylated, alkylated or phosphorylated (e.g., compounds in which the amino group on the compound used in the invention has been eicosanoylated, alanylated, bentylaminoaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated, or tert-butylated); in cases where the compound used in the agent of the present invention includes a hydroxyl group, compounds in which the hydroxyl group has been acylated, alkylated, phosphorylated or borated (e.g., compounds in which the hydroxyl group on the compound used in the invention has been acetylated, palmitoylated, propanoylated, pivaloylated, succinylated, fumarylated, aranylated or dimethylaminomethylcarbonylated); and, in cases where the compound used in the agent of the present invention includes a carboxyl group, compounds in which the carboxyl group has been esterified or amidated (e.g., compounds in which the carboxyl group on the compound used in the invention has been ethyl esterified, phenyl esterified, carboxymethyl esterified, dimethylaminomethyl esterified, pivaloyloxymethyl esterified, 1-{(ethoxycarbonyl)oxy}ethyl esterified, phthalidyl esterified, (5-methyl-2-oxo-1,3-dioxolen-4-yl)methyl esterified, 1-{[(cyclohexyloxy)carbonyl]oxylethyl esterified or methyl amidated). These compound may be manufactured by known methods. Prodrugs of the compound used in the agent of the present invention may be either hydrates or non-hydrates. Alternatively, the prodrugs of compounds used in the agent of the present invention may be compounds which change to the compound used in the agent of the present invention under physiological conditions, such as those mentioned in Iyakuhin no Kaihatsu (The development of medicines), Vol. 7: Bunshi Sekkei (Molecular design) (Hirokawa Shoten, 1999), pp. 163-198). Moreover, the compound used in the agent of the present invention may be labeled with an isotope (e.g., $^3H$, $^{14}C$, $^{35}S$, $^{125}I$).

Method for Preparing EP1 Agonist

The EP1 agonist used in the agent of the present invention may be prepared by a method described in, for example, Japanese Patent Application Laid-open No. H11-322709, WO 99/02165, WO 00/51585, or WO 00/51616, or by a method in general accordance therewith. For example, the (13E)-(11α,15S,17S)-2,5-ethano-6,9-dioxo-11,15-dihydroxy-17,20-dimethylprosta-13-enoic acid mentioned herein as an example of a preferred EP1 agonist may be prepared by the method described in Japanese Patent Application Laid-open No. H11-322709.

Toxicity

The toxicity of the EP1 agonist used in the agent of the present invention was confirmed to be very low, making it safe enough for use as a pharmaceutical agent.

Application to Pharmaceutical Products

Because EP1 agonists have an immunopotentiating effect mediated by cytotoxic T lymphocyte activation and/or natural killer cell (NK1.1+ cell) activation, they are useful for the prevention and/or treatment of cancers, microbial infectious diseases and the like in mammals (e.g., humans and non-human animals (e.g., monkeys, sheep, cattle, horses, dogs, cats, rabbits, rats and mice). When used concomitantly with the subsequently described antigen peptides, EP1 agonists may also serve as a sensitizer or adjuvant for the antigen peptide.

That is, EP1 agonists (or combinations of an EP1 agonist and a subsequently described antigen peptide) are useful for treating various diseases caused by microbial infections. Examples of such diseases include cellulitis, acute lymphangitis, lymphadenitis, skin abscesses, necrotizing subcutaneous infections, staphylococcal scalded skin syndrome, folliculitis, furunculosis, suppurative hidradenitis, carbuncles, infectious paronychia, erythrasma, dermatophyte infections (e.g., ringworm), candidiasis, tinea versicolor, scabies, crippling disease, epidemic typhus, murine typhus, scrub typhus, Rocky Mountain spotted fever, ehrlichiosis, rickettsialpox, bartonellosis, verrucosis, acute respiratory diseases, acute pharyngoconjunctival fever, acute keratoconjunctivitis, shingles, Ramsay Hunt syndrome, progressive multifocal leukoencephalopathy and arboviral encephalitis.

Antigen Peptide

The agent of the present invention may further include as an ingredient therein an antigen peptide, or may be used concomitantly with an antigen peptide as a sensitizer or adjuvant therefore.

In cases where an antigen peptide is used as an ingredient of the agent of the present invention, or in cases where an antigen peptide is used concomitantly with the agent of the present invention, it is preferable to employ an antigen which enables cCTLs to specifically recognize the cancer or microorganism serving as the target of immunopotentiation by the agent of the present invention. An example would the use of tyrosinase-related protein 2 (TRP-2), a type of melanoma-specific antigen peptide, as the antigen peptide in anticipation of immunopotentiation against melanoma.

Other antigen peptides include, for example, cancer (tumor) antigen peptides and virus antigen peptides. Examples of cancer (tumor) antigen peptides include MAGE-1, MAGE-2, MAGE-3, MAGE-A4, MAGE-6, MART1, TRP-1, tyrosinase, gp100, HER2/neu, CEA, β-catenin, CHP, CpG, MUC-1, NY-ESO-1, BAGE, GAGE-1, GAGE-2, SAGE, LAGE, WT-1, hTERT, CDK4, p15, p53, PSA, gp1001, MAGE-12, telomerase, SART, SYT-SSX, survivin, CTL precursor-oriented peptide, MN/CA9, OY-TES-1, SCP-1, GnT-V and PRAME. Examples of virus antigen peptides include EB virus antigen, Cytomegarovirus antigens, herpesvirus antigens (e.g., HSV glycoprotein B), influenza virus antigens (e.g., influenza virus nucleprotein (NP)-derived peptide, M1 peptide, M2 peptide, HA peptide, NA peptide, PB1 peptide, PB2 peptide, PA peptide, NP2 peptide), and HIV antigens. Examples of other antigen peptides include Salmonella antigens, dysentery antigens, Enterobacter antigens, and protozoa-derived antigens and parasite-derived antigens.

The above-mentioned cancer (tumor) antigen peptides, as will be apparent to those skilled in the art, may be selectively used according to the cancer to be treated and the specific major histocompatibility antigen (human leukocyte antigen (HLA)) which binds with the antigen peptide. By way of illustration, for the treatment of melanoma, antigen peptide which binds with HLA-A0201 or HLA-A2402 may be used; specifically, the use of MART-1, gp-100, MAGE-2, MAGE-3, tyrosinase or TRP-2 is preferred.

Likewise, with regard to viral antigen peptides as well, in cases where treatment is targeted at influenza virus infections, the use of influenza virus NP peptide is preferred.

The above-mentioned antigen peptides, as will be apparent to those skilled in the art, are preferably partial sequences corresponding to epitopes which are specifically recognized by CTLs. For example, VYDFFVWL (SEQ ID NO: 1) is used as a partial sequence of TRP-2. Similarly, NP366-374: ASNENMETM (SEQ ID NO: 2), NP55-69 and NP147-158 may be used as partial sequences of influenza virus NP peptide. In the same way, it is possible to use, for example, M2e-1 peptide, M2e-2 peptide and M2e-3 peptide as partial sequences of the influenza virus M2 peptide. And use may be made of, for example, HA91-108 as a partial sequence of the influenza virus HA peptide.

In cases where the agent of the present invention includes an antigen peptide, of the various types of antigen peptides mentioned above, any one type may be included alone, or two or more such types may be used together in any combination and ratio thereof.

Other Drugs

Aside from the above-mentioned antigen peptides, the agent of the present invention may include other drugs as ingredients, or such other drugs may be administered concomitantly, so as to (1) complement and/or strengthen the immunopotentiating effect; (2) improve the kinetics and absorption, and reduce the administered dose, of the EP1 agonist; and/or (3) reduce the side effects of the EP1 agonist.

Such other drugs may be low-molecular-weight compounds, or may be, for example, high-molecular-weight proteins, polypeptides, polynucleotides (DNA, RNA, genes), antisense oligonucleotides, decoy compounds, antibodies or vaccines (exclusive of the aforementioned antigen peptides). Such other drugs include not only ones that have been discovered to date, but include also ones that will be discovered in the future.

The other drugs are exemplified by immunostimulants, anticancer agents (e.g., alkylating agents, antimetabolites, anticancer antibiotics, plant-based preparations, hormonal drugs, platinum compounds, histone deacetylase (HDAC) inhibitors, poly(ADP-ribose) polymerase (PARP) inhibitors), antiviral agents, antibiotics, antifungal agents, antiparasitic agents and antiprotozoal agents.

Examples of immunostimulants include lentinan, picibanil, krestin, sizofiran, ubenimex, interferon, lobenzarit, TF, GM-CSF, M-CSF, G-CSF, IL-1, IL-2, IL-3 and IL-12.

Examples of alkylating agents include nitrogen mustard-N-oxide hydrochloride, cyclophosphamide, iphosphamide, melphalan, thiotepa, carboquone, busulfan, nimustine hydrochloride, dacarbazine and ranimustine.

Examples of antimetabolites include methotrexate, mercaptopurine, 6-mercaptopurine riboside, fluorouracil, tegafur, tegafur/uracil, carmofur, doxifluridine, cytarabine, enocitabine, tegafur/gimestat/potassium otastat, gemcitabine hydrochloride, cytarabine ocfosfate, procarbazine hydrochloride and hydroxycarbamide.

Examples of anticancer antibiotics include actinomycin D, mitomycin C, daunorubicin hydrochloride, doxorubicin hydrochloride, aclarubicin hydrochloride, neocarcinostatin, pirarubicin hydrochloride, epirubicin (hydrochloride), idarubicin hydrochloride, chromomycin A3, bleomycin (hydrochloride), peplomycin sulfate, therarubicin and zinostatin/stimalamer.

Examples of plant-based preparations include vinblastine sulfate, vincristine sulfate, vindesine sulfate, irinotecan hydrochloride, etoposide, flutamide, vinorelbine tartrate, docetaxel hydrate and paclitaxel.

Examples of hormonal drugs include estramustine phosphate sodium, mepitiostane, epitiostanol, goserelin acetate, fosfestrol (diethylstilbestrol phosphate), tamoxifen citrate, toremifene citrate, fadrozole hydrochloride hydrate, medroxyprogesterone acetate, bicalutamide, leuprorelin acetate, anastrozole and exemestane.

Examples of platinum compounds include carboplatin, cisplatin and nedaplatin.

Examples of HDAC inhibitors include vorinostat, AN-9, belinostat, MGCD-0103, MS-275, panobinostat, romidepsin, tacedinaline, valproic acid, VP-101, CRA-024781, ITF-2357, pyroxamide, CS-055, EHT-0205, FR-135313, NSC-3852, PXD-118490, SAHA analogs and LAQ-824.

Examples of PARP inhibitors include GPI-15427, GPI-16539, GPI-18078, GPI-6000, GPI-6150, KU-0687, INO-1001, FK-866, 4-(4-(N,N-dimethylaminomethyl)phenyl)-5-hydroxyisoquinolinone, FR-255595, FR-257516, FR-261529, FR-247304, M-50916, ABT-472, ONO-1924H, DR-2313, CEP-8983, AG-014699, BGP-15, AAI-028, PD-141076, PD-141703 and ONO-2231.

Examples of antiviral agents include anti-HIV agents (e.g., CCR5 antagonist, CXCR4 antagonist, reverse transcriptase inhibitors, fusion inhibitors), antiinfluenza viral agents (e.g., oseltamivir phosphate, zanamivir hydrate), antiherpesvirus agents (e.g., acyclovir), interferon-$\alpha$ or $\beta$, and various types of immunoglobulins.

Examples of antibiotics include cephem antibiotics (e.g., cefaclor), penicillin antibiotics (e.g., amoxicillin), macrolide antibiotics (e.g., erythromycin ethylsuccinate), ceftibuten, cefuroxime sodium, doxorubicin, tobramycin, meropenem trihydrate, cefetamet pivoxil hydrochloride, astromicin sulfate, sisomycin sulfate and netilmicin sulfate.

Examples of antifungal agents include itraconazole, fluconazole, lanoconazole, sulconazole nitrate, oxiconazole nitrate, econazole nitrate, itoconazole nitrate, croconazole nitrate, clotrimazole, terbinafine hydrochloride, tolnaftate, bifonazole, neticonazole hydrochloride, ketoconazole, butenafine hydrochloride, miconazole nitrate, voriconazole, amphotericin B, flucytosine, griseofulvin and micafungin.

Examples of antiparasitic agents include santonin, combantrin, spatonin, mebendazole, mintezol, eskazole, biltricide, quinine, fansidar, fragile and fasigyne.

Examples of antiprotozoal agents include metronidazole and pentamidine.

When the agent of the present invention includes another drug, of the various types of other drugs mentioned above, any one type may be included alone, or two or more such types may be used together in any combination and ratio thereof.

Other Ingredients

In addition to the above-mentioned EP1 agonists, and the above-mentioned antigen peptides and/or other

drugs that may be optionally used, the agent of the present invention may also include other ingredients as well. Examples of such other ingredients include pharmaceutical bases such as excipients, binders, disintegrants, lubricants and stabilizers. These pharmaceutical bases are subsequently described.

Method of Using the agent of the present invention

The agent of the present invention, as described above, is administered to a subject such a mammal for the purpose of immunopotentiation thereof. When the agent of the present invention is used for the above purpose, it will generally be administered orally or parenterally, and either systemically or locally.

The dose of the agent of the present invention is determined so as to include an effective amount of EP1 agonist for producing the desired immunopotentiating effect in the subject. The effective dose of EP1 agonist will vary according to, for example, the type of subject, age, body weight, symptoms, therapeutic efficacy, method of administration, period of administration, and other immunotherapies to be administered together. In cases where the subject is human, for a single administration in one adult having a body weight of 70 kg, the dose is generally in a range of at least 0.1 ng, preferably at least 1 ng, and more preferably at least 10 ng, but generally not more than 1,000 mg, preferably not more than 100 mg, and more preferably not more than 10 mg. Of course, as noted above, because the effective dose and the administered dose will vary depending on various conditions, there will be cases in which administration in a smaller amount than the above range suffices, and there will be cases in which administration in a large amount exceeding the above range is required.

The mode of administration for the agent of the present invention will vary accordingly to, for example, the type of subject, age, body weight, symptoms, therapeutic efficacy, method of administration and period of administration. For example, a single dose of the agent of the present invention containing the above-indicated effective dose of EP1 agonist may be orally administered from one to several times per day, or may be parenterally administered from one to several times per day. Alternatively, the agent of the present invention may be continuously administered intravenously for a period ranging from one hour to 24 hours per day, or may be continuously administered locally for a period ranging from one day to three months.

When the agent of the present invention is administered, it may be used as a solid or liquid preparation for oral administration, or it may be used as an injection for parenteral administration, as an external preparation, as a suppository, as eye drops, as an inhalant or the like.

External preparations for parenteral administration include such dosage forms as ointments, gels, creams, wet packs, adhesive preparations, liniments, mists, inhalants, sprays, eye drops, ear drops and nasal drops. These may include one or more active ingredient, and may be manufactured and prepared by known methods or commonly used formulations.

Ointments may be manufactured by a known method or a commonly used formulation. For example, the ointment may be manufactured or prepared by triturating or melting one or more active ingredient (EP1 agonist, and also antigen peptide and/or other drug which may be optionally used) in a base. The ointment base may be selected from among known ointment bases or substances commonly used for this purpose. Illustrative examples include higher fatty acids and higher fatty acid esters (e.g., adipic acid, myristic acid, palmitic acid, stearic acid, oleic acid, adipic acid esters, myristic acid esters, palmitic acid esters, stearic acid esters, oleic acid esters), waxes (e.g., beeswax, spermaceti, ceresin), surfactants (e.g., polyoxyethylene alkyl ether phosphate), higher alcohols (e.g., cetanol, stearyl alcohol, cetostearyl alcohol), silicone oils (e.g., dimethylpolysiloxane), hydrocarbons (e.g., hydrophilic petrolatum, white petrolatum, refined lanolin, liquid paraffin), glycols (e.g., ethylene glycol, diethylene glycol, propylene glycol, polyethylene glycol, macrogol), vegetable oils (e.g., castor oil, olive oil, sesame oil, turpentine), animal oils (e.g., mink oil, egg yolk oil, squalane, squalene), water, absorption promoters and anti-irritants. Any one of these may be selected and used alone, or two or more may be used in admixture. The ointment may also include, for example, humectants, preservatives, stabilizers, antioxidants and fragrances.

Gels may be manufactured by a known method or a commonly used formulation. For example, the gel may be manufactured or prepared by melting one or more active ingredient in a base. The gel base may be selected from among known gel bases or substances commonly used for this purpose. Illustrative examples include lower alcohols (e.g., ethanol, isopropyl alcohol), gelling agents (e.g., carboxymethyl cellulose, hydroxyethyl cellulose, hydroxypropyl cellulose, ethyl cellulose), neutralizing agents (e.g., triethanolamine, diisopropanolamine), surfactants (e.g., polyethylene glycol monostearate), gums, water, absorption promoters and anti-irritants. Any one of these may be selected and used alone, or two or more may be used in admixture. The gel may also include, for example, preservatives, antioxidants and fragrances.

Creams may be manufactured by a known method or a commonly used formulation. For example, the cream may be manufactured or prepared by melting or emulsifying one or more active ingredient in a base. The cream base may be selected from among known cream bases or substances commonly used for this purpose. Illustrative examples include higher fatty acid esters, lower alcohols, hydrocarbons, polyhydric alcohols (e.g., propylene glycol, 1,3-butylene

glycol), higher alcohols (e.g., 2-hexyl decanol, cetanol), emulsifying agents (e.g., polyoxyethylene alkyl ethers, fatty acid esters), water, absorption promoters and anti-irritants. Any one of these may be selected and used alone, or two or more may be used in admixture. The cream may also include, for example, preservatives, antioxidants and fragrances.

Wet packs may be manufactured by a known method or a commonly used formulation. For example, the wet pack may be manufactured by melting one or more active ingredient in a base, then spreading and coating the mixture onto a backing. The wet pack base may be selected from among known wet pack bases or substances commonly used for this purpose. Illustrative examples include thickeners (e.g., polyacrylic acid, polyvinyl pyrrolidone, gum arabic, starch, gelatin, methyl cellulose), wetting agents (e.g., urea, glycerol, propylene glycol), fillers (kaolin, zinc oxide, talc, calcium, magnesium), water, solubilizing agents, tackifiers and anti-irritants. Any one of these may be selected and used alone, or two or more may be used in admixture. The wet pack may also include, for example, preservatives, antioxidants and fragrances.

Adhesive preparations may be manufactured by a known method or a commonly used formulation. For example, the adhesive preparation may be manufactured by melting one or more active ingredient in a base, then spreading and coating the mixture onto a backing. The adhesive preparation base may be selected from among known adhesive preparation bases or substances commonly used for this purpose. Illustrative examples include high-molecular-weight bases (e.g., styrene-isoprene-styrene block copolymer, polyisobutylene rubber, acrylate resin, acrylic copolymer resins, silicone rubbers), oils and fats, higher fatty acids, transcutaneous penetration promoters (e.g., oleic acid, isopropyl myristate, D-menthol, crotamiton), tackifiers (e.g., rosin derivatives, alicyclic saturated hydrocarbon resins), and anti-irritants (e.g., glycerol, crotamiton). Any one of these may be selected and used alone, or two or more may be used in admixture. The adhesive preparation may also include, for example, preservatives, antioxidants and fragrances. Examples of adhesive preparations include plasters (e.g., matrix (adhesive single layer)-type adhesive preparations, reservoir-type adhesive preparations), and poultices. In addition, matrix-type adhesive preparations include drug-dispersing matrix-type adhesive preparations and drug dissolving matrix-type adhesive preparations. Plasters are also called tapes.

Liniments may be manufactured by a known method or a commonly used formulation. For example, the liniment is manufactured or prepared by dissolving, suspending or emulsifying one or more active ingredient in one or more selected from among water, alcohols (e.g., ethanol, polyethylene glycol), higher fatty acids, glycerol, soap, emulsifying agents and suspending agents. The liniment may also include, for example, preservatives, antioxidants and fragrances.

Mists, inhalants and sprays, aside from a commonly employed diluent, may also include a stabilizer such as sodium bisulfate and/or a buffer agent that imparts isotonicity, examples of which include tonicity agents such as sodium chloride, sodium citrate or citric acid. These may also be prepared as aerosols.

Inhalants for parenteral administration include aerosols, dusts for inhalation, and liquids for inhalation. Such liquids for inhalation may be in a form used by dissolution or suspension in water or some other suitable solvent at the time of use.

These inhalants are prepared in accordance with a known method.

For example, a liquid for inhalation is prepared by the suitable selection of the following, as needed: a preservative (e.g., benzalkonium chloride, paraben), colorant, buffering agent (e.g., sodium phosphate, sodium acetate), tonicity agent (e.g., sodium chloride, concentrated glycerol), thickener (e.g., carboxyvinyl polymer), and an absorption promoter.

A powder for inhalation is prepared by suitable selection of the following, as needed: a lubricant (e.g., stearic acid and salts thereof), binders (e.g., starch, dextrin), excipients (e.g., lactose, cellulose), colorants, preservatives (e.g., benzalkonium chloride, paraben), and an absorption promoter.

A conventional atomizer or nebulizer is used when administering a liquid for inhalation, and a conventional inhaler for powder preparations is used when administering a powder for inhalation.

Injections for parenteral administration encompass solutions, suspensions, emulsions, and solid injections which are dissolved or suspended in a solution at the time of use. An injection may be used after dissolving, suspending or emulsifying one or more active ingredient in a solvent. Examples of solvents that may be used include distilled water for injection, physiological saline, vegetable oils, propylene glycol, polyethylene glycol, alcohols such as ethanol, and combinations thereof. Such injections may also include stabilizers, solubilizers (e.g., glutamic acid, aspartic acid, Polysorbate 80 (registered trademark)), suspending agents, emulsifiers, analgesics, buffering agents and preservatives. These may be sterilized in the final step, or production and preparation may be carried out by aseptic manipulation. Alternatively, a sterile solid preparation, such a lyophilized product, may be produced, and this may be used by dissolution in distilled water for injection or some other solvent which is either sterilized before use or is aseptic.

Other compositions for parenteral administration include suppositories for rectal administration and vaginal suppositories for vaginal administration which include one or more active ingredient and are formulated by a conventional method.

Solid preparations for internal use via oral administration include tablets, pills, capsules, powders and pellets. Capsules include hard capsules and soft capsules. Tablets include sublingual tablets, oral adhesive tablets and oral rapidly disintegrating tablets.

Such solid preparations for internal use are employed after production by a conventional method from one or

more active ingredient, either alone or in admixture with an excipient (e.g., lactose, mannitol, glucose, microcrystalline cellulose, starch), binder (e.g., hydroxypropyl cellulose, polyvinyl pyrrolidone, magnesium aluminometasilicate), disintegrant (e.g., calcium cellulose glycolate), lubricant (e.g., magnesium stearate), stabilizer, and solubilizer (e.g., glutamic acid, aspartic acid). If necessary, one or more layer may be coated thereon using a coating agent (e.g., sucrose, gelatin, hydroxypropyl cellulose, hydroxypropylmethyl cellulose phthalate). Moreover, the active ingredient may be used by being included within a capsule composed of a substance such as gelatin that is capable of being absorbed.

The sublingual tablets are manufactured and prepared in accordance with a known method. For example, one or more active ingredient is mixed with an excipient (e.g., lactose, mannitol, glucose, microcrystalline cellulose, colloidal silica, starch), binder (e.g., hydroxypropyl cellulose, polyvinyl pyrrolidone, magnesium aluminometasilicate), disintegrant (e.g., starch, L-hydroxypropyl cellulose, carboxymethyl cellulose, croscarmellose sodium, calcium cellulose glycolate), lubricant (e.g., magnesium stearate), swelling agent (e.g., hydroxypropyl cellulose, hydroxypropylmethyl cellulose, carbopol, carboxymethyl cellulose, polyvinyl alcohol, xantham gum, guar gum), swelling aid (e.g., glucose, fructose, mannitol, xylitol, erythritol, maltose, trehalose, phosphates, citrates, silicates, glycine, glutamic acid, arginine), stabilizer, solubilizer (e.g., polyethylene glycol, propylene glycol, glutamic acid, aspartic acid) and flavor (e.g., orange, strawberry, mint, lemon, vanilla), manufactured as a preparation according to a conventional method and used. If necessary, one or more layer may be coated thereon using a coating agent (e.g., sucrose, gelatin, hydroxypropyl cellulose, hydroxypropylmethyl cellulose phthalate). Commonly used additives such as preservatives, antioxidants, colorants and sweeteners may be added where necessary.

Oral adhesive tablets are manufactured and prepared according to a known method. For example, one or more active ingredient is mixed with an excipient (e.g., lactose, mannitol, glucose, microcrystalline cellulose, colloidal silica, starch), binder (e.g., hydroxypropyl cellulose, polyvinyl pyrrolidone, magnesium aluminometasilicate), disintegrant (e.g., starch, L-hydroxypropyl cellulose, carboxymethyl cellulose, croscarmellose sodium, calcium cellulose glycolate), lubricant (e.g., magnesium stearate), adhesive (e.g., hydroxypropyl cellulose, hydroxypropylmethyl cellulose, carbopol, carboxymethyl cellulose, polyvinyl alcohol, xantham gum, guar gum), adhesion aid (e.g., glucose, fructose, mannitol, xylitol, erythritol, maltose, trehalose, phosphates, citrates, silicates, glycine, glutamic acid, arginine), stabilizer, solubilizer (e.g., polyethylene glycol, propylene glycol, glutamic acid, aspartic acid) and flavor (e.g., orange, strawberry, mint, lemon, vanilla), manufactured as a preparation according to a conventional method and used. If necessary, one or more layer may be coated thereon using a coating agent (e.g., sucrose, gelatin, hydroxypropyl cellulose, hydroxypropylmethyl cellulose phthalate). Commonly used additives such as preservatives, antioxidants, colorants and sweeteners may be added where necessary.

Oral rapidly disintegrating tablets are manufactured and prepared according to a known method. For example, one or more active ingredient, either alone or in the form of a powder or granulated powder particles coated with a suitable coating agent (e.g., ethyl cellulose, hydroxypropyl cellulose, hydroxypropylmethyl cellulose, acrylic acid-methacrylic acid copolymer) or with a plasticizer (e.g., polyethylene glycol, triethyl citrate) is mixed with an excipient (e.g., lactose, mannitol, glucose, microcrystalline cellulose, colloidal silica, starch), binder (e.g., hydroxypropyl cellulose, polyvinyl pyrrolidone, magnesium aluminometasilicate), disintegrant (starch, L-hydroxypropyl cellulose, carboxymethyl cellulose, croscarmellose sodium, calcium cellulose glycolate), lubricant (e.g., magnesium stearate), dispersing aid (e.g., glucose, fructose, mannitol, xylitol, erythritol, maltose, trehalose, phosphates, citrates, silicates, glycine, glutamic acid, arginine), stabilizer, solubilizer (e.g., polyethylene glycol, propylene glycol, glutamic acid, aspartic acid) and flavor (e.g., orange, strawberry, mint, lemon, vanilla), manufactured as a preparation according to a conventional method and used. If necessary, one or more layer may be coated thereon using a coating agent (e.g., sucrose, gelatin, hydroxypropyl cellulose, hydroxypropylmethyl cellulose phthalate). Commonly used additives such as preservatives, antioxidants, colorants and sweeteners may be added where necessary.

Liquid preparations for internal use via oral administration include pharmaceutically allowable solutions, suspensions, emulsions, syrups and elixirs.
In such liquid preparations, one or more active ingredient is dissolved, suspended or emulsified in a commonly used diluent (e.g., purified water, ethanol, or a mixture thereof). Moreover, these liquid preparations may include, for example, wetting agents, suspending agents, emulsifying agents, sweeteners, flavoring agents, fragrances, preservatives and buffering agents.

In cases where an antigen peptide and/or another drug is used in addition to the EP1 agonist, these may both be used as ingredients of the agent of the present invention and administered in the form of a combination preparation obtained by combining both ingredients within a single preparation, or some or all of the antigen peptide and/or other drug may take a form which is administered as a separate preparation from the agent of the present invention.

In cases where the antigen peptide and/or other drug takes a form which is administered as a separate preparation from the agent of the present invention, such preparations may be administered at the same time as the agent of the present invention or may be administered with a time difference therebetween. When administered with a time difference therebetween, the agent of the present invention, antigen peptide and other drugs are not subject to any particular limitation in the order of administration thereof, and may be administered in any order.

The doses in which the antigen peptide and/or other drug are administered, both in cases where these serve as ingredients of the agent of the present invention and in cases where they are rendered into preparations separate from the agent of the present invention, may be suitably selected based on the clinically used doses. Moreover, the relative proportions of the EP1 agonist used in the agent of the present invention and the antigen peptide and/or other drugs may be suitably selected according to such factors as the age and body weight of the subject in which they are to be administered, the method of administration and the period of administration. For example, the antigen peptide and/or other drug may be used in an amount of from 0.01 to 100 parts by weight per part by weight of the EP1 agonist used in the agent of the present invention.

Immunotherapy

In order to increase the immunopotentiating effects of the agent of the present invention (e.g., the immunopotentiating effects against cancers, microbial infectious diseases, etc.), concomitant use may be made of other commonly used immunotherapies. Examples of other than immunotherapies include any immunotherapies for cancers, microbial infectious diseases and the like, for example, transcutaneous immunotherapies, (e.g., tape stripping), natural killer (NK) cell therapy, CTL therapy (herein, CTL therapy means immune cell therapy using cultured cancer-specific CTLs from an outside source), cytokine therapy, CD3-LAK therapy, LAK therapy, and dendritic cell therapy. Moreover, the agent of the present invention may be used concomitantly with another commonly used cancer treatment modality or microbial infectious disease treatment modality. Specific examples of such cancer treatment modalities include chemotherapy using the above-mentioned anticancer drugs, radiation therapy, particle (charged heavy particle) radiotherapy, stereotactic irradiation, thermal therapy and hematopoietic stem cell transplantation. Of these immunotherapies and cancer treatment modalities, transcutaneous immunotherapy, particularly tape stripping, is especially preferred for use together with the agent of the present invention.

The tape stripping refers to a method of administering a substance (the EP1 agonist, antigen peptide, etc. used in the agent of the present invention) which activates cytotoxic T lymphocytes at sites where the corneal layer (of the skin epidermis) has been physically or chemically removed using a adhesive tape preparation, acetone or the like, as described in, for example, Japanese Patent No. 3879785. Herein, it is preferable to use the adhesive tape preparation. Specific examples of preferred tape preparations include those wherein an acrylic polymer, rubber-based polymer, hydrophilic polymer or the like is used as the adhesive, and a plastic film (e.g., polyethylene, polyethylene terephthalate, polyurethane, polyethylene, polypropylene, polyester, polyvinyl acetate, ethylene-vinyl acetate copolymer) is used as the backing. Methods for destroying the corneal layer using the adhesive tape preparation are exemplified by methods in which the operation of stripping the tape preparation after affixing it to the surface of the corneal layer of the epidermis is repeated from one to several times.

When the agent of the present invention is used concomitantly with the above-mentioned tape stripping method, the EP1 agonist used in the agent of the present invention may be administered by including it in the adhesive layer of the tape preparation used in tape stripping, it may be administered by any method (e.g., adhesive preparation, injection) to the site where the corneal layer (of the skin epidermis) has been stripped by tape stripping, or it may be administered outside of this site (e.g., by oral administration, intravenous administration, subcutaneous administration). Also, when an antigen peptide is concomitantly used, the antigen peptide may be administered by inclusion in the adhesive layer of the tape or by inclusion in the adhesive layer together with the EP1 agonist, it may be administered by any method (e.g., adhesive preparation) to the above-described tape stripping site, or it may be administered outside of this site (e.g., by intravenous administration).

The method of administering the antigen peptide is exemplified by a method which entails, following destruction of the corneal layer, having a gauze pad of any size absorb a solution of the antigen peptide (e.g., in dimethylsulfoxide (DMSO) or in a phosphate buffer solution (PBS)), then affixing the pad to one or a plurality of sites where the corneal layer has been deprived. In cases where the antigen peptide is administered to a plurality of sites, it may be administered in different sites on the arms, thighs, abdomen and back. The period of administration is exemplified by continuous administration for one to three months during which the pads are changed every 24 hours.

In order to effectively elicit the immunopotentiating effects of the agent of the present invention (particularly the immunopotentiating effects on cancers or microbial infectious diseases, and most particularly the immunopotentiating effect on melanoma or an influenza virus), it is preferable to combine as indicated below the above-mentioned EP1 agonists, antigen peptides and other modalities for treating cancers or microbial infectious diseases. That is, it is preferable to combine (13E)-(11α,15S,17S)-2,5-ethano-6,9-dioxo-11,15-dihydroxy-17,20-dimethylprosta-13-enoic acid as the EP1 agonist with an antigen peptide, it is more preferable to combine (13E)-(11α,15S,17S)-2,5-ethano-6,9-dioxo-11,15-dihydroxy-17,20-dimethylprosta-13-enoic acid with an antigen peptide and another immunotherapy for cancer or a microbial infectious disease, and it is especially preferable to combine (13E)-(11α,15S,17S)-2,5-ethano-6,9-dioxo-11,15-dihydroxy-17,20-dimethylprosta-13-enoic acid with a melanoma-specific antigen peptide (TRP-2) or an influenza virus-specific antigen peptide (influenza virus NP peptide) and the tape stripping.

The above combination may be used as a pharmaceutical or a pharmaceutical composition in immunotherapy for melanoma or influenza viruses using the tape stripping. In a preferred aspect, an adhesive preparation containing the EP1 agonist (13E)-(11$\alpha$,15S,17S)-2,5-ethano-6,9-dioxo-11,15-dihydroxy-17,20-dimethylprosta-13-enoic acid as the active ingredient in the adhesive layer is applied to the corneal layer of the skin epidermis, or corneal layer, that has been stripped by the tape stripping. Moreover, when using such an adhesive preparation, it is preferable to concomitantly use the antigen peptide TRP-2 or influenza virus NP peptide. In the present aspect, these active ingredients may be administered as separate adhesive preparations, although administration as an adhesive preparation which includes both (13E)-(11$\alpha$,15S,17S)-2,5-ethano-6,9-dioxo-11,15-dihydroxy-17,20-dimethylprosta-13-enoic acid and the antigen peptide TRP-2 or influenza virus NP peptide within the adhesive layer thereof is more preferred.

In the above-described pharmaceutical or pharmaceutical composition (particularly adhesive preparation), the systemic circulation of the drug can be avoided by local administration to the skin, thus making it possible to reduce side effects that could conceivably occur due to systemic circulation from oral administration, intravenous administration or the like.

The present invention includes also cytotoxic T lymphocytes activated by EP1 agonists. Such cells may be produced by treating immature T cells cultured by a known technique with an optimal amount of EP1 agonist. The presence or absence of cytotoxic T lymphocyte activation can be analyzed by a commonly used method, such as flow cytometric analysis. The cells obtained in this way may be used in immunotherapy, such as CTL therapy.

## EXAMPLES

The following experiments serve to demonstrate that EP1 agonists exhibit the effects of the invention. However, the experimental methods shown below are not to be construed as limiting the scope of the present invention. By way of illustration, it is possible to evaluate the immunopotentiating effects on target cancers and microbial infectious diseases by carrying out the same procedure using the antigen peptides listed above instead of the antigen peptide TRP-2 and influenza virus NP peptide used in the following examples.

### Example 1: Activation of Cytotoxic T Lymphocytes in Cervical Lymph Nodes

Example 1(1): The corneal layer of the auricles of C57BL/6 (B6) mice was destroyed by repeatedly carrying out (ten times) a stripping operation using adhesive tape, following which an antigen peptide or EP1 agonist was applied as a test substance. Using a solution obtained by dissolving a melanoma-specific antigen peptide (available from Peptide Business Department, Accord K.K.; abbreviated below as TRP-2) as the antigen peptide in 70% ethanol, 10 $\mu$g of the antigen peptide was applied per side of an auricle. (13E)-(11$\alpha$,15S,17S)-2,5-Ethano-6,9-dioxo-11,15-dihydroxy-17,20-dimethylprosta-13-enoic acid (abbreviated below as Compound A) as the EP1 agonist was dissolved in an acetone/olive oil = 10:1 mixed solvent to form a 100 $\mu$M solution, of which 20 $\mu$L was applied per side of an auricle. One week after application of the test substance, the cervical lymph nodes were removed. The TRP-2-specific CTL frequency, relative to an arbitrary value of 100 representing the total number of mononucleocytes in the cervical lymph nodes, was determined by flow cytometric analysis (FACScaliber; Becton, Dickinson and Company) with a tetramer (Medical & Biological Laboratories Co., Ltd.). Four experimental groups were established: (1) a group in which only tape stripping (abbreviated below as TS) was carried out, (2) a group in which TRP-2 was applied after TS, (3) a group in which Compound A was applied after TS, and (4) a group in which TRP-2 and Compound A were applied after TS.

It should be noted that the melanoma-specific antigen peptide TRP-2 was synthesized based on the partial sequence shown below and mentioned in Non-patent Document 1 (Peptide Business Department, Accord K.K.). TRP-2: tyrosinase-related protein 2 181-188
Sequence: Val Tyr Asp Phe Phe Val Trp Leu (SEQ ID NO: 1)

Results:

The results are shown in Table 1 below.

Table 1

| Experimental group | (1) | (2) | (3) | (4) |
|---|---|---|---|---|
| Ratio of TRP-2-specific CTLs | 0.13 | 0.44 | 0.53 | 0.68 |

In the groups in which Compound A was applied as the EP1 agonist (Experimental groups (3) and (4)), the ratio of TRP-2-specific CTLs in the cervical lymph nodes increased relative to Experimental Group (1), suggesting that

the EP1 agonist has a TRP-2-specific CTL activating effect. The results obtained in Experimental Group (4) suggest that this effect increases with use of the EP1 agonist in combination with an antigen peptide.

Example 1(2): Using NP peptide (abbreviated below as NP), an influenza virus-specific antigen, as the antigen peptide, the same procedure was carried out as in Example 1(1), and the test compound was applied to C57BL/6 (B6) mice. Five days after application of the test substance, cervical lymphocytes were collected in each experimental group from the removed cervical lymph nodes. The collected spleen cell lymphocytes and the NP were cultured together in an IL-2 (10 U/mL) containing culture medium, thereby amplifying the NP-specific CTLs. In a separate procedure, murine thymoma-derived EL4 cells as the target cells were labeled with calcein (Dojindo), and both an NP-pulsed EL4 cell group exposed to 10 μg/mL of NP and an EL 4 cell group not subjected to NP pulsing were established. The cultured NP-specific CTLs and calcein-labeled EL4 cells were mixed, and the number of calcein-labeled EL-4 cells remaining after 3 hours was determined using a Terascan (Minerva Tech K.K.). The following groups were established as experimental groups: (1) a group in which only TS was carried out, (2) a group in which NP was applied after TS, and (3) a group in which NP and Compound A were both applied after TS. The number of EL4 cells were determined for each experimental group, both with and without NP pulsing. Specific lysis (%) was computed by a conventional method using the following formula.

$$\text{Specific lysis (\%)} = \{1 - (\text{EL4 cell count})\} \times 100$$

The partial sequence shown in SEQ ID NO: 2 (Nature, Vol. 348, pp. 252-254 (1990)) was used as the influenza virus-specific antigen peptide NP (Medical & Biological Laboratories Co., Ltd. (MBL)).
Influenza virus A/PR/8/34 strain; influenza virus nucleoprotein (NP) 366-374
Sequence: Ala Ser Asn Glu Asn Met Glu Thr Met (SEQ ID NO: 2)

Results:

The results for Example 1(2) are shown in Table 2 below. The values indicate the specific lysis (%).

Table 2

| Experimental group | (1) | (2) | (3) |
|---|---|---|---|
| EL4 cells not subjected to NP pulsing | 1.2 | 1.2 | 5 |
| NP-pulsed EL4 cells | 1.8 | 11.9 | 28.5 |

The above results indicate that when an antigen peptide is administered (Experimental Group (2)), only the EL4 cells which express NP are specifically attacked, which indicates that NP-specific CTLs are being induced. Moreover, the results in the group in which EP1 agonist was administered at the same time (Experimental Group (3)) suggest that the concomitant use of an EP1 agonist and an antigen peptide significantly increases the ability to induce NP-specific CTLs.

Example 2: Activation of Cytotoxic T Lymphocytes in Isolated Langerhans Cells

An epidermal cell suspension was prepared by trypsinizing an auricle epidermal sheet removed over a given surface area from the corneal layer on one of the auricles in C57BL/6 (B6) mouse. The resulting epidermal cell suspension was subjected to panning using an antibody specific for I-$A^b$ antigen in order to isolate epidermal Langerhans cells. Each test substance (TRP-2 (concentration, 10 μg/mL) and Compound A (1 μM)) was added to the isolated Langerhans cells (cell density, $10^5$ cells/mL), following which the cells were cultured in a culture medium (cRPMI1640 medium; Sigma-Aldrich Japan). After a half-day, the cells were then co-cultured for 7 days with spleen cell lymphocytes isolated from the cervical lymph nodes of B6 mice. The frequency of TRP-2-specific CTLs, relative to an arbitrary value of 100 representing the total number of mononucleocytes in the culture medium, was subsequently determined by flow cytometric analysis (FACScaliber; Becton, Dickinson and Company) with a tetramer (Medical & Biological Laboratories Co., Ltd.). The following groups were established as experimental groups: (1) an untreated group, (2) a group in which TRP-2 was added, and (3) a group in which TRP-2 and Compound A were both added.

Results:

The results are shown in Table 3 below.

Table 3

| Experimental group | (1) | (2) | (3) |
|---|---|---|---|
| Ratio of TRP-2-specific CTLs | 2.1 | 3.2 | 8.8 |

In the group in which TRP-2 was added as the antigen peptide (Experimental Group (2)), the ratio of TRP-2-specific CTLs among the B6 spleen cell lymphocytes increased, demonstrating that TRP-2-specific CTLs were induced. Furthermore, the results from the group in which EP1 agonist was added at the same time (Experimental Group (3)) suggest that the ability to induce TRP-2-specific CTLs is boosted by the concomitant use of an EP1 agonist and an antigen peptide.

Example 3: Changes in Expression of Cell-Surface Molecules in Cultured Langerhans Cells

Using the same procedure as in Example 2 above, the Langerhans cells of C57BL/6 (B6) mice were isolated, following which Compound A (1 μM) was added as an EP1 agonist and the cells were cultured for a half-day in a culture medium (cRPMI1640 medium; Sigma-Aldrich Japan). The cultured cells were then stained using antibodies specific to the cell-surface molecules I-$A^b$, B7-1 (CD80), B7-2 (CD86), CD40 and ICAM-1 (CD54), and changes in the expression of these molecules were analyzed by flow cytometry. An experimental group treated in the same way, excluding the addition of Compound A, was established as a control.

Results:

The results are shown in Table 4 below.

Table 4

| Cell-surface molecule | Expression of each cell-surface molecule (%) | |
|---|---|---|
| | Control group | Compound added group |
| I-$A^b$ | 12.1 | 28.3 |
| B7-1 | 5.9 | 8.8 |
| B7-2 | 65.5 | 65.2 |
| CD40 | 28.7 | 30.8 |
| ICAM-1 | 36.5 | 46.3 |

These results demonstrate that, in Langerhans cells, the EP1 agonist increases the expression of not only I-$A^b$, which is an MHC class II antigen molecule, but also of B7-1 and ICAM-1 molecules, which are T cell co-stimulatory molecules.

Example 4: Melanoma Growth-Inhibiting Effect

Test substances (TRP-2 and Compound A) were applied to a site where the corneal layer of the right auricle in B6 mice had been removed by TS in the same way as in Example 1. Two weeks later, the same treatment was carried out on the left auricle. The same combination of test substances was applied to both ears, with TRP-2 being applied in an amount of 10 μg per side of an auricle and Compound A being applied as 20 μL of a 100 μM solution per side of an auricle. Four days after the above treatments had been carried out, $2\times10^5$ B16 melanoma cells (RIKEN Cell Bank) were subcutaneously transplanted into the mice, and tumor growth was examined. Three experimental groups were established (each group being composed of 4 animals): (1) a group in which only TS was carried out, (2) a group in which TRP-2 was applied after TS, and (3) a group in which TRP-2 and Compound A were applied after TS.

Results:

The tumor diameters (mm) in each group were measured at given intervals (days) following B16 melanoma cell inoculation. The results are shown in FIG. 1.

In the group in which Compound A was applied as the EP1 agonist (Experimental Group (3)), the tumor diameters were small and a decrease in the rate of tumor growth was observed relative to Experimental Group (1). This effect, given that the tumor growth rate decreased markedly compared with TRP-2 (Experimental Group (2)), suggests that the combined use of EP1 agonist and antigen peptide markedly boosts the melanoma growth-inhibiting effect of the antigen peptide.

Example 5: Life-Prolonging Effects in Melanoma-Inoculated Mice

B16 melanoma cells ($2\times10^5$) were transplanted into B6 mice in the same way as in Example 4. On day 5 after transplantation, the respective test substances (TRP-2 (10 μg per side of an auricle), Compound A (20 μL of a 100 μM solution per side of an auricle)) were administered to the right auricles in B6 mice by the same method as in Example 2. The same treatment was carried out ten days later on the left auricle, following which the survival of the mice in each experimental group was examined. The following groups were established as experimental groups (each group being composed of 8 animals): (1) a group in which only TS was carried out, (2) a group in which compound A was applied after TS, and (3) a group in which TRP-2 and Compound A were applied after TS.

Results:

The change over time in the number of survivors in each experimental group is shown in Table 5.

Table 5

| Number of days after inoculation of B16 melanoma cells | Experimental Group (1) | Experimental Group (2) | Experimental Group (3) |
|---|---|---|---|
| 14 | 8 | 8 | 8 |
| 16 | 8 | 8 | 8 |
| 24 | 8 | 8 | 8 |
| 25 | 8 | 8 | 8 |
| 28 | 8 | 8 | 8 |
| 30 | 6 | 8 | 8 |
| 32 | 2 | 8 | 8 |
| 34 | 2 | 8 | 8 |
| 36 | 0 | 4 | 8 |
| 38 | 0 | 0 | 8 |
| 41 | 0 | 0 | 8 |
| 44 | 0 | 0 | 6 |
| 46 | 0 | 0 | 6 |
| 50 | 0 | 0 | 4 |
| 52 | 0 | 0 | 4 |
| 55 | 0 | 0 | 4 |
| 56 | 0 | 0 | 4 |

The fact that all the animals in Experimental Group (1) had died by day 36, whereas a number of animals remained alive on day 36 in the groups given EP1 agonist indicates that EP1 agonist has a life-prolonging effect in melanoma-inoculated mice. Striking life-prolonging effects were observed in Experimental Group (3) in particular, with half of the animals remaining alive even on day 56 after transplantation. This suggests that the concomitant use of EP1

agonist and antigen peptide increases the life-prolonging effect.

Example 6: Activation of NK1.1+ cells in Cervical Lymph Nodes

As in Example 1, the corneal layer of the auricles of B6 mice was destroyed by repeatedly stripping ten times using adhesive tape, following which an EP1 agonist (Compound A: 20 μL of a 100 μM solution per side of an auricle) was applied as the test substance. Two weeks after application of the test substance, the cervical lymph nodes were removed. The frequency of NK1.1+ cells, relative to an arbitrary value of 100 representing the total number of mononucleocytes in the cervical lymph nodes, was determined by flow cytometric analysis. The following groups were established as experimental groups: (1) a group in which only TS was carried out, and (2) a group in which Compound A was applied after TS.

Results:

The results are shown in Table 6 below.

Table 6

| Experimental Group | (1) | (2) |
|---|---|---|
| Ratio of NK1.1+ cells | 2.7 | 3.6 |

In the group in which Compound A was applied as the EP1 agonist (Experimental Group (2)), the ratio of NK1.1+ cells in the cervical lymph nodes increased relative to Experimental Group (1)), suggesting that the EP1 agonist has a NK1.1+ cell activating effect.

Example 7: Splenic Lymphocyte Proliferative Ability Test

As in Example 6, TS was carried out, after which Compound A (20 μL of a 100 μM solution per side of an auricle) was applied as the substance. One week after application of the test substance, splenic lymphocytes were recovered, then cultured at 37°C for 3 days in a culture medium (cRPMI1640 medium: Sigma-Aldrich Japan) containing IL-2 (10 U/mL). Cell proliferative ability tests were carried out using WST-1 (Takara Bio Inc.) as the cell proliferation reagent (absorbance at 450 nm). Experimental groups similar to those in Example 6 were established (with each group containing three animals).

Results:

The results are shown in Table 7 below. The relative values of the absorption intensities in each experimental group, based on an arbitrary value of 1 for the absorbance at 450 nm when all of the WST-1 added had decomposed to formazan, indicate the percent uptake of WST-1; that is, the cell proliferation ratio.

Table 7

| Experimental Group | (1) | (2) |
|---|---|---|
| Uptake of WST-1 (%) | 4.2 | 27.3 |

In the group where Compound A was applied as the EP1 agonist (Experimental Group (2)), the cell proliferation ratio increased relative to Experimental Group (1)), suggesting that the EP1 agonist has a splenic lymphocyte proliferative ability.

Preparation Examples

Preparation Example 1: Tablets

An ethanol solution (1,000 mL) of (13E)-(11α,15S,17S)-2,5-ethano-6,9-dioxo-11,15-dihydroxy-17,20-dimethylprosta-13-enoic acid (300 mg), magnesium stearate (10 g), silicon dioxide (2,000 mg), talc (1,000 mg) and calcium cellulose glycolate (20 g) were mixed by a conventional method and dried, following which microcrystalline cellulose (500 g) was added, the total weight was brought up to 1,000 g and the ingredients were thoroughly mixed until uniform.

The mixture was then formed into tablets by a conventional process, giving 10,000 tablets containing 30 μg of active ingredient per tablet.

Preparation Example 2: Injection

(13E)-(11α,15S,17S)-2,5-Ethano-6,9-dioxo-11,15-dihydroxy-17,20-dimethylprosta-13-enoic acid (50 mg) was dissolved in distilled water for injection (30 L), and the solution was sterilization filtered with a membrane filter, following which 3 mL of the sterilized solution was filled into individual 5 mL ampules for injection, thereby giving an injection containing 5 μg of active ingredient per ampule (10,000 ampules).

Preparation Example 3: Adhesive Preparation

A styrene-isoprene-styrene block copolymer (300 mg), colorless rosin ester (300 mg) and light liquid paraffin (400 mg) were dissolved in 1,000 mg of ethyl acetate (Kishida Chemical Co., Ltd.) to prepare a viscous solution. Next, (13E)-(11α,15S,17S)-2,5-ethano-6,9-dioxo-11,15-dihydroxy-17,20-dimethylprosta-13-enoic acid (40 g) was dissolved in the viscous solution to prepare a coating solution. The coating solution was spread onto a backing to a thickness of about 60 μm using a Baker applicator. The resulting tacky surface was vacuum-dried at room temperature for 18 hours. The dried tacky surface was covered with a release liner and cut as appropriate, thereby producing an adhesive preparation (main ingredient content, 0.2 mg/$cm^2$).

INDUSTRIAL APPLICABILITY

EP1 agonists have an immunopotentiating effect mediated by cytotoxic T lymphocyte activation and/or natural killer cell activation. Accordingly, they are useful in the prevention and/or treatment of cancers, microbial infectious diseases and the like.

**Claims**

1. An immunopotentiating agent comprising an EP1 agonist.

2. The immunopotentiating agent according to claim 1, wherein the EP1 agonist is a 6-oxo-$PGE_1$ compound represented by general formula (I)

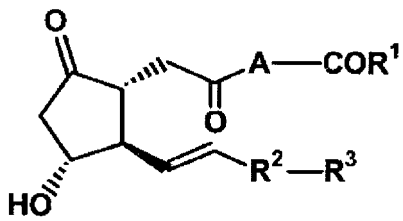

(I)

(wherein A is a carbon ring of 4 to 7 members;
$R^1$ is hydroxyl, $C_{1-4}$ alkoxy or $NR^4R^5$ ($R^4$ and $R^5$ being each independently a hydrogen atom or $C_{1-4}$ alkyl);
$R^2$ is $C_{1-8}$ alkylene, $C_{3-8}$ alkenylene or $C_{3-8}$ alkynylene substituted with one hydroxyl;
$R^3$ is

(1) a hydrogen atom or $C_{1-4}$ alkyl,
(2) phenyl or $C_{3-7}$ cycloalkyl which may be substituted with 1 to 3 substituents selected from among $C_{1-4}$ alkyl, $C_{1-4}$ alkoxy, halogen atoms, trifluoromethyl and nitro, or
(3) phenoxy which may be substituted with 1 to 3 substituents selected from among $C_{1-4}$ alkyl, $C_{1-4}$ alkoxy, halogen atoms, trifluoromethyl and nitro;

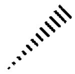

is an α-configuration bond; and

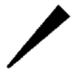

is a β-configuration bond, with the proviso that (i) when $R^2$ is a $C_{3-8}$ alkenylene or $C_{3-8}$ alkynylene substituted with one hydroxyl, the hydroxyl is not bonded to a carbon atom associated with a double bond or triple bond, and (ii) when $R^3$ is (3), the hydroxyl on $R^2$ and the $R^3$ radical are not bonded to the same carbon atom), or a salt or cyclodextrin clathrate thereof.

**3.** The immunopotentiating agent according to claim 1, wherein the EP1 agonist is used for potentiating the immune reaction to a cancer and/or a microbial infectious disease.

**4.** The immunopotentiating agent according to claim 3, wherein the cancer is one or more selected from among a digestive organ cancer, a skin cancer, a respiratory cancer, a urogenital cancer, a liver cancer and a pancreatic cancer.

**5.** The immunopotentiating agent according to claim 4, wherein the skin cancer is melanoma.

**6.** The immunopotentiating agent according to claim 3, wherein the microorganism is one or more selected from among a virus, a bacterium and a fungus.

**7.** The immunopotentiating agent according to claim 6, wherein the virus is an influenza virus.

**8.** The immunopotentiating agent according to claim 2, wherein the EP1 agonist is (13E)-(11α,15S,17S)-2,5-ethano-6,9-dioxo-11,15-dihydroxy-17,20-dimethylprosta-13-enoic acid.

**9.** The immunopotentiating agent according to claim 1, further comprising an antigen peptide.

**10.** The immunopotentiating agent according to claim 8, wherein the antigen peptide is a melanoma-specific antigen peptide.

**11.** A pharmaceutical composition for potentiating immune reaction to melanoma, comprising (13E)-(11α,15S,17S)-2,5-ethano-6,9-dioxo-11,15-dihydroxy-17,20-dimethylprosta-13-enoic acid as an active ingredient and optionally including a melanoma-specific antigen peptide.

**12.** A method for potentiating immune reaction in a mammal, comprising administering an effective dose of an EP1 agonist to the mammal.

**13.** Use of an EP1 agonist for preparing an immunopotentiating agent, which is optionally used in combination with an antigen peptide.

**14.** An EP1 agonist for potentiating immune reaction, which is optionally used in combination with an antigen peptide.

Fig.1

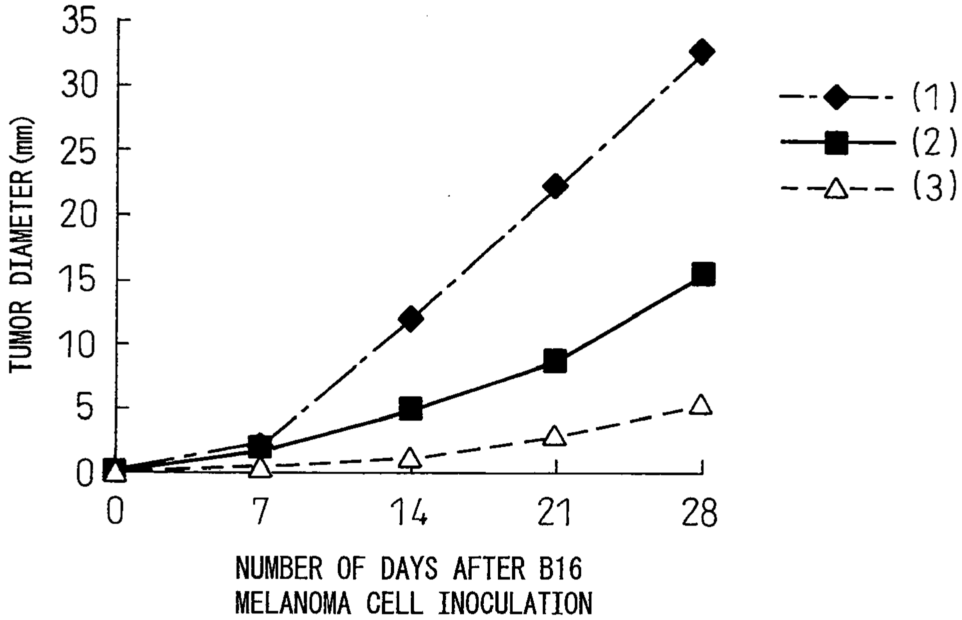
35
30
25
20
15
10
5
0
TUMOR DIAMETER (mm)
0
7
14
21
28
NUMBER OF DAYS AFTER B16
MELANOMA CELL INOCULATION
(1)
(2)
(3)

**INTERNATIONAL SEARCH REPORT**

International application No.
PCT/JP2009/058305

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
A61K45/00, A61K31/5575, A61K38/00, A61K39/00, A61K39/39, A61K47/40, A61P31/00, A61P31/04, A61P31/10, A61P31/12, A61P35/00, A61P37/04, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus/REGISTRY(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | Daiji Sakata et. al., Facilitation of Th1-mediated immune response by prostaglandin E receptor EP1, The 81st Annual Meetings of The Japanese Pharmacological Society, 2008.02.15, 94P 02H1-1 | 1-11,13 |
| Y | Daiji Sakata et. al., PGE2-EP1 signaling facilitate Th1 immune response, The 80th Annual Meeting of The Japanese Pharmacological Society, 2007.02.28, 55P E1H4-3 | 1-11,13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>10 June, 2009 (10.06.09) | Date of mailing of the international search report<br>23 June, 2009 (23.06.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2009/058305

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-510038 A (Schering Corp.), 18 March, 2003 (18.03.03), Claim 23; Par. No. [0015] & US 2003/0162261 A1 & EP 1210434 A & WO 2001/018051 A2 & DE 60038304 D & AU 7360800 A & CA 2388562 A & HU 301102 A & IL 148300 D & CN 1387571 A & AT 389019 T & & ES 2300276 T & HK 1044170 A | 3-7,9-11,13 |
| X<br>Y | WO 2002/76505 A1 (Ono Pharmaceutical Co., Ltd.), 03 October, 2002 (03.10.02), Claims & US 2004/0122100 A1 & EP 1374899 A1 & DE 60214987 D & CA 2440710 A & AT 340577 T | 14<br>1-11,13 |
| X<br>Y | JP 11-322709 A (Ono Pharmaceutical Co., Ltd.), 24 November, 1999 (24.11.99), Claims; Par. No. [0008]; example 1 (Family: none) | 14<br>1-11,13 |
| Y | JP 2002-512202 A (Arch Development Corp.), 23 April, 2002 (23.04.02), Claims & US 6080399 A & EP 1071450 A & WO 1999/053949 A2 & AU 3865999 A & CA 2326406 A & NZ 507829 A | 9-11,13 |

Form PCT/ISA/210 (continuation of second sheet) (April 2007)

**INTERNATIONAL SEARCH REPORT**

International application No.
PCT/JP2009/058305

Continuation of A. CLASSIFICATION OF SUBJECT MATTER
(International Patent Classification (IPC))

*A61K45/00*(2006.01)i, *A61K31/5575*(2006.01)i, *A61K38/00*(2006.01)i, *A61K39/00*(2006.01)i, *A61K39/39*(2006.01)i, *A61K47/40*(2006.01)i, *A61P31/00*(2006.01)i, *A61P31/04*(2006.01)i, *A61P31/10*(2006.01)i, *A61P31/12*(2006.01)i, *A61P35/00*(2006.01)i, *A61P37/04*(2006.01)i, *A61P43/00*(2006.01)i

(According to International Patent Classification (IPC) or to both national classification and IPC)

Form PCT/ISA/210 (extra sheet) (April 2007)

**INTERNATIONAL SEARCH REPORT**

International application No.
PCT/JP2009/058305

**Box No. II Observations where certain claims were found unsearchable (Continuation of item 2 of first sheet)**

This international search report has not been established in respect of certain claims under Article 17(2)(a) for the following reasons:

1. [X] Claims Nos.: 12
because they relate to subject matter not required to be searched by this Authority, namely:
Claim 12 pertains to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

INTERNATIONAL SEARCH REPORT

International application No.
PCT/JP2009/058305

Claims 1, 3-7, 9, 10, 13 and 14 relate to a composition comprising a substance defined by a desired property "an EP1 agonist" as an active ingredient.

These claims include compositions of all of the substances having the property. However, it appears that those which are supported by the description in the meaning within PCT Article 6 and disclosed in the meaning within PCT Article 5 are limited to an extremely small part of the claimed substances.

Further, even though the common technical knowledge at the time of filing the present application is taken into consideration, the scope of the substance having the property "an EP1 agonist" cannot be specified. Therefore, these claims do not comply with the requirement of clearness under PCT Article 6, either.

Because some of substances of interest can be obtained merely by screening does not mean that the broad scope of the invention including the substances defined by the function or property can become clear, or because the results of a pharmacological test on some "EP1 agonists" are described does not also mean that the compositions of the compound specified by its function and including an infinite number of various components within its scope are fully supported by the description.

Such being the case, in this search report, the search was made on the substance specified in claim 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3879785 B
- WO 0069465 A
- JP H11322709 B
- WO 9902165 A
- WO 0051585 A
- WO 0051616 A
- JP S503362 B
- JP S5231404 B
- JP S6152146 B


### Non-patent literature cited in the description


- *Proceedings of the Natural Academy Sciences of USA,* 2000, vol. 97, 371-376
- *Cancer Research,* 2006, vol. 66, 10136-10144
- *Neoplasia,* 2001, vol. 3, 402-410
- *The Journal of Experimental Medicine,* 2007, vol. 204, 2865-2874
- Iyakuhin no Kaihatsu. Bunshi Sekkei. Hirokawa Shoten, 1999, vol. 7, 163-198
- *Nature,* 1990, vol. 348, 252-254